Europäisches Patentamt

(19) European Patent Office

Office européen des brevets

(11) Publication number : **0 491 895 B1**

# (12) EUROPEAN PATENT SPECIFICATION

(45) Date of publication of patent specification :
**19.07.95 Bulletin 95/29**

(51) Int. Cl.⁶ : **G01N 33/84, C12Q 1/56**

(21) Application number : **91911692.1**

(22) Date of filing : **21.06.91**

(86) International application number :
**PCT/US91/04405**

(87) International publication number :
**WO 92/01229 23.01.92 Gazette 92/03**

(54) FACTOR VIII:Ca CHROMOGENIC ASSAY.

(30) Priority : **12.07.90 US 553219**

(43) Date of publication of application :
**01.07.92 Bulletin 92/27**

(45) Publication of the grant of the patent :
**19.07.95 Bulletin 95/29**

(84) Designated Contracting States :
**BE CH DE ES FR GB IT LI NL**

(56) References cited :
Scandinavian Journal of Haemotology, Supplement no. 40, vol. 33, 1984, (Copenhagen, DK), S. Rosen: "Assay of factor VIII:C with a chromogenic substrate", pages 139-145, see the whole article
Haemostasis, vol. 17, no. 1-2, January-April 1987, Karger AG, (Basel, CH), G. Van Dieijen et al.: "Spectrophotometric method for the assay of human blood coagulation factor VIII", pages 14-24, see the whole article
Thrombosis Research, vol. 10, no. 2, 1977, Pergamon Press, (GB), H. Suomela et al.: "The activation of factor X evaluated by using synthetic substrates", pages 267-281, see the whole article (cited in the application)
The Journal of Biological Chemistry, vol. 256, no. 7, 10 April 1981, (US), G. Van Dieijen et al.: "The role of phospholipid and factor VIIIa in the activation of bovine factor X", pages 3433-3442, see the whole article
Biochemistry, vol. 24, no. 27, 31 December 1985, American Chemical Society, (Washington, US), P. Lollar et al.: "Activation of porcine factor VIII:C by thrombin and factor Xa", pages 8056-8063, see the whole document

(56) References cited :
Biochemistry, vol. 19, no. 3, 5 February 1980, American Chemical Society, (Washington, US), G.A. Vehar et al.: "Preparation and properties of bovine factor VIII (antihemophilic factor)", pages 401-410, see the whole article

(73) Proprietor : **BAXTER DIAGNOSTICS INC.**
**One Baxter Parkway**
**Deerfield, IL 60015 (US)**

(72) Inventor : **HEMKER, Hendrik, Coenraad**
**Tongersestraat 41**
**NL-6211 LM Maastricht (NL)**
Inventor : **KESSELS, Hendricus, Johannes**
**Papenstraat 6**
**NL-6211 LM Maastricht (NL)**
Inventor : **BEGUIN, Suzette, Lucette**
**Akerstraat 12 B**
**NL-6211 LG Maastricht (NL)**

(74) Representative : **Bassett, Richard Simon et al**
**ERIC POTTER & CLARKSON**
**St. Mary's Court**
**St. Mary's Gate**
**Nottingham NG1 1LE (GB)**

## Description

This invention relates generally to the field of chromogenic assays and more specifically to a chromogenic assay for the determination of levels of the blood coagulation factor VIII:Ca contained in a sample, e.g. plasma.

Background of the Invention

Clot formation in plasma is initiated by the serial enzymatic activation of clotting factors, which results in the appearance of small traces of thrombin. Hurlet-Birk Jensen A., et al. Factor V and VIII Activation "In Vivo" During Bleeding. Evidence of Thrombin Formation at the Early Stage of Hemostasis. Path. Biol. 1976; 24, p.6-10. The explosive nature of the coagulation process is a consequence of the position feedback reactions that these thrombin traces exert on the cofactors V. Colman, RW, The Effect of Proteolytic Enzymes on Bovine Factor VI. Kinetics of Activation and Inactivation by Bovine Thrombin, Biochem. 1969; 4: p.1438-1444, Lindhout, MJ, Activation of Bovine Factor V by Thrombin and a Protease from Russell's Viper Venom (RVV), Thromb. Haemost. 1979; 42: p.491. Rapaport, SI, et al., Further Evidence That Thrombin Activation of Factor VIII is an Essential Step in Intrinsic Clotting, Scand J. Clin. Lab. Invest. 1965; 17: p.84-88, Biggs, R., et al. Thrombin and the Interaction of Factors VIII and IX, Brit J. Haemat. 1965; 11: p.276-295, Hemker, HC, et al., Reaction Sequence of Blood Coagulation, Nature 1967; 215: p.1201, Osterud, B., et al., Formation of Intrinsic Factor X Activator With Special Reference to the Role of Thrombin, Br. J. Haematol 1971; 21: p.643-660, Hultin, MB, et al., Activation of Factor X by Factors IXa andVIII; A Specific Assay for Factor IXa in the Presence of Thrombin-Activated Factor VIII, Blood 1978; 52: p.928-940, and on platelets, Davey, MG, et al., Actions of Thrombin and Other Proteolytic Enzymes on Blood Platelets, Nature 1967; 216: p.857-858. Activated Factors V and VIII:C dramatically boost the performance of Factors Xa and IXa, whereas activated platelets provide, among other things, the negatively charged surface necessary for most coagulation reactions. Bevers, EM, et al., Generation of Prothrombin Converting Activity and the Exposure of Phosphatidylserine at the Outer Surface of Platelets, Eur. J. Bioch. 1982; 122: p.429-436.

According to prior methods Factor VIII:C concentrations, and also Factor VIII:C activation in plasma were measured using coagulation assays, involving VIII:C deficient plasma. Soulier, P., et al., Deficit En Beme Facteur Prothromboplastique plasmatique, Rapports Entre le PTA et le Facteur Hageman, Thrombin. Diathes. Haemorrh. 1958; 2: p.1, Rappaport, SI, et al., A Simple Specific One-Stage Assay for Plasma Thromboplastin Antecedent Activity, J. Lab. Clin. Med. 1961; 57: p.771, Hardisty, RM, et al., A One-Stage Factor VIII (Antihemophilic Globulin) Assay and Its Uses on Venous and Capillary Plasma, Thrombos. Diathes. Haemorrh. 1962; 7: p.215, Veltkamp, JJ, et al., Detection of the Carrier State in Hereditary Coagulation Disorders, Thrombos. Diathes. Haemorrh. 1968; 19: p.279-303 and 403-422, Suomela, H, et al. The Activation of Factor X Evaluated by Using Synthetic Substrates, Thromb. Res. 1977; 1: p.267-281, Van Dieijen, G, et al., The Role of Phospholipid and Factor VIIIa in the Activation of Bovine Factor X, J. Biol. Chem. 1981; 256: p. 3433-3442. The occurrence of various feedback reactions, however, made it impossible to relate, in a quantitatively reliable way, these values to the amounts of activated VIII:Ca. The advent of a chromogenic substrate for Factor Xa, and the recognition that a property of activated Factor VIII:C is accelerated activation of Factor X by IXa, phospholipids and calcium ions made a more direct method of determining functional Factor VIII:Ca conceivable. Suomela, H, et al. Supra, Van Dieijen, et al., The Role of Phospholipid and Factor VIIIa in the Activation of Bovine Factor X, J. Biol. Chem. 1981; 256: p.3433-3442. Still, feedback reactions of Factor Xa on Factor VIII:C, and the inactivation of Factor Xa by the antithrombin III and α 1-antitrypsin present in plasma, threatened to seriously hamper the feasibility of such a Factor VIII:Ca assay in plasma. Vehar, GA, et al., Preparation and Properties of Bovine Factor VIII (Antihemophilic Factor), Biochemistry 1980; 19: p.401-410, Hultin, MB, Role of Human Factor VIII in Factor X Activation, J.Clin. Invest. 1982; 69: p.950-955, Lollar, P, et al., Activation of Porcine Factor VIII:C by Thrombin and Factor Xa, Biochemistry 1985; 24: p.8056-8064, Neuenschwanter, P, et al., A Comparison of Phospholipid and Platelets in the Activation of Human Factor VIII by Thrombin and Factor Xa, and in the Activation of Factor X, Blood 1988; 72: p.1761-1770, and Factor X, Jesty, J., et al., The Mechanism of Activation of Factor X, J.Biol.Chem. 1974; 249: p.5614-5622. A Factor VIII assay is described in Haemostasis 1987; 17 (1-2); p.14-24 where all of the Factor VIII in the sample is purposely firstly converted into its activated form, and the latter is then assayed without distinguishing the amount of Factor VIIIa present in the sample before the conversion step. On the basis of the observation that *in situ* generated thrombin is the only enzyme that effectively activates factor VIII:C and factor V in plasma, we developed a scheme for measuring Factor VIII:Ca in plasma, that circumvents these difficulties.

The classical method for the determination of Factor VIII:Ca activity in plasma estimates the activity of the clotting factor in a plasma sample from the amount of time by which it shortens the prolonged clotting time of a plasma congenitally deficient in that factor, as compared to a normal plasma. As there is no solid theoretical basis that underlies the relationship between the coagulation time and the percentage of coagulation factor activity, these assays are bioassays in the sense that the activities have to be read from a standard curve, and that the values of these activities are only valid with respect to the method by which they are measured. Consequently, a need exists for a more quantitative method to measure Factor VIII:Ca.

## Summary of the Invention

This invention provides a highly sensitive, reproducible, and convenient assay for determination of the levels of blood coagulation Factor VIII:Ca contained in blood serum, plasma, and other fluids. Since Factor VIII:Ca is not an enzyme, its functional concentration cannot be measured directly by way of a chromogenic substrate. Instead, the ability of Factor VIII:Ca to enhance Factor X activation in the presence of Factor IXa, phospholipids, and $Ca_{2+}$, is exploited. Factor Xa concentration can be determined chromogenically, which permits a quantitative estimation of the Factor-VIII:Ca a level.

In the assay of this invention, a test sample of blood serum, plasma, or other Factor VIII:Ca containing fluid is added to a solution containing activated blood coagulation Factor IXa, and calcium ions and phospholipid. Factor X is added to the mixture. Factor IXa and Factor VIII:Ca act to accelerate the conversion of the Factor X, a zymogen, to activated Factor X, (hereafter referred to as Factor Xa). An indicator agent is added to the reaction mixture, which reacts with the Factor Xa so formed, to release a signal molecule, which may be conveniently measured. The following equations further illustrate the steps of the present method:

$$2) \quad \text{Factor X} \quad \xrightarrow[\substack{\text{Factor IXa} \\ \text{Phospholipid} \\ Ca^{++}}]{\text{Factor VIII:Ca}} \quad \text{Factor Xa}$$

$$3) \quad \text{Indicator Agent} \quad \xrightarrow{\text{Factor Xa}} \quad \text{signal molecule}$$

In accordance with the method of this invention, an assay is provided which has a high degree of sensitivity and reproducibility for Factor VIII:Ca concentration. This invention also provides a kit for the convenient performance of routine laboratory assays of Factor VIII:Ca containing fluids. Preferably, the assay is not affected by the presence of heparin and other blood clot interactive substances. Preferably, a bulk source of assay components to facilitate the operation of automated equipment capable of processing for assay large numbers of test samples. It is an object of this invention to suppress side reaction of Factor Xa that might distort the correlation. To that end it was found that inhibition of these side reactions can be obtained by using a high concentration of chromogenic substrate for Factor Xa.

This assay methodology may be applicable to the measurement of blood coagulation enzymes containing other activated factors; in particular e.g. Factor V. An activating enzyme (Enzyme A, i.e. the Factor IXa - factor VIII:Ca - phospholipid complex), is measured by indirectly measuring the rate of activation of an enzyme that is its natural substrate. The enzyme that results from this activation (Enzyme B) is allowed to act immediately on an indicator molecule. By its reaction with the indicator molecule (the concentration of which must suffice to saturate enzyme B) complicating reactions involving enzyme B are inhibited. (i.e. example, the activation of Factor VIII:C by Factor Xa, and the inhibition of Factor Xa by antithrombin III). Because enzyme B increases linearly in time, the signal molecule is produced according to a parabolic curve. The slope of the first derivative of this parabola is proportional to the concentration of enzyme A.

The advantages and performance of the present invention will be better understood by reference to the following detailed description and Example.

## Detailed Description of the Preferred Embodiment

The method of the assay of the present invention comprises the steps of:
1. Combining a sample and a solution containing a sufficient amount of Factor IXa to saturate substantially all Factor VIII:Ca in said sample; a sufficient amount of a thrombin inhibitor to inhibit thrombin activity without affecting Factor Xa activity, a sufficient amount of phospholipids, calcium ion to facilitate the conversion of Factor X to Factor Xa.
2. Adding to the mixture a sufficient amount of Factor X to saturate substantially all of the complex of Factor IXa, Factor VIII:Ca and phospholipid.

3. Adding to the mixture a sufficient amount of an indicator agent capable of reacting with Factor Xa, said quantity of indicator agent being sufficient to suppress Factor Xa side reactions, to release a signal molecule; and

4. Measuring the signal molecule. The foregoing method may be exemplified by reference to the following equations:

$$1) \quad \text{Factor X} \quad \xrightarrow[\substack{\text{Phospholipid} \\ \text{Ca}^{++}}]{\substack{\text{Factor VIII:Ca} \\ \text{Factor IXa}}} \quad \text{Factor Xa}$$

$$2) \quad \text{Indicator Agent} \xrightarrow{\text{Factor Xa}} \text{signal molecule}$$

In practicing the method of the present invention, Factors IX and X may be obtained from virtually any animal or human source, and may be prepared by any fractionation or concentration method known to the art. In addition, a highly purified source of such factors is from recombinant vectors propagated in suitable host cell lines. One advantage to using factors from animal or recombinant vector sources is assurance that the product factors will not be contaminated with human pathogens such as hepatitis A and B, HTLV-III, or other such viruses. In the preferred embodiment of the present method, blood coagulation factors are of bovine origin.

It should be noted that because the sample undergoes a considerable dilution in the assay mixture (30 to 100 fold), the endogenous Factor IX concentration will very probably not be high enough to saturate all Factor VIII:Ca (up to 1 U/ml) present in the sample on Factor IXa, which is necessary for a reliable assay. Thus, a sufficient amount of Factor IXa must be added.

The conversion of Factor X to Xa proceeds most efficiently in the presence of phospholipids. These phospholipids may be such representative compounds as phosphotidyl choline, phosphotidyl serine, or cholesterol and mixtures thereof in various proportions. Other lipid and phospholipid compositions may be substituted as well.

It is our experience that optimal results are obtained with vesicles of 20 mole-% phosphatidyl serine and 80 mole-% phosphatidyl choline. This is not critical however; the ranges of acceptable composition are 5 to 40 mole-% phosphatidyl serine, 1 to 20 mole-% cholesterol, and 50-90 mole-% of phosphotidyl choline.

Any chemical source of calcium cation may be used to effectuate the conversion of Factor X. Sufficient calcium ion may be added to the original incubation mixture to drive the reaction converting Factor X to Factor Xa, or a second amount of calcium ion may be added at the time Factor X is to be converted. While the source of calcium cation ($Ca^{++}$) may be $CaCl_2$, $Ca(NO_2)_2$, $CaSO_4$, or other inorganic calcium cation containing compounds, the preferred source is $CaCl_2$.

A thrombin inhibitor is used to block thrombin activity on chromogenic substrate for Factor Xa. Useful thrombin inhibitors include α-NAPAP and hirudin.

In performing the assay of this invention, great variations in protein concentrations, incubation times, reagent concentrations, and temperatures may be employed. The selection of particular assay parameters will be influenced by the source, type, and size of the sample to be assayed, the anticipated levels of Factor VIII:Ca contained therein, and the threshold of sensitivity desired. Taking these circumstances into account, selection of assay parameters will be apparent to those skilled in the art. The parameters of the assay, which will enable anyone skilled in the art to carry out the assay in accordance with a preferred embodiment are set forth in the Example which follows.

It should be noted that, although the rate of Factor X activation is linearly proportional to the level of Factor VIII:Ca, the rate of amidolysis of the chromogenic substrate is not. In fact, the relation between absorbance and Factor VIII:Ca concentration is a second order polynomial. Because of this, at least two points are needed for a determination of the Factor VIII:Ca concentration. We found that best results are obtained when absorbance is measured continuously in time. The resulting curve is then analyzed by way of a second order least squares fit procedure. This yields the parameters A, B and C (equation: $A + B.t + C.t^2$). C is proportional to Factor VIII:Ca concentration.

Accordingly, an optional additional step in the present assay consists of adding a quenching agent to the incubation mixture at a fixed point in time after commencement of the reaction converting Factor X to Factor Xa. The quenching composition may be any substance capable of disrupting a protein-mediated chemical reaction, but the preferred composition is a buffered solution comprised of Tris, ethylenediaminetetracetic acid, sodium chloride, and sodium azide.

The blood coagulation factors of the present assay and the Factor VIII:Ca protein to be assayed are fragile functional proteins, and desirably a stabilizing substance or substances may be included during the incubation to optimize assay conditions and to protect functionality of assay components. Such stabilizing substances also protect functionality during storage wherein the assay components are maintained in either a wet or lyophilized state. Various stabilizers are known in the art; the preferred substances being polyethylene glycol and bovine serum albumin, either singly or in combination.

The indicator agent of the present invention is a molecule capable of reacting with blood coagulation Factor Xa. In such reaction, by-products of chemical reaction must be generated which produce a measurable signal moiety, U.S. Patent Nos. 4,480,030 and 4,666,831 describe a class of chromogenic compounds capable of reacting with Factor Xa. The indicator of formula $CH_3OCO-D-CHG-Gly-Arg-pNA-AcOH$ wherein (see US-A-4 480 030, column 7) D-CHG is D-2-cyclohexylglycine and pNA is p-nitroanilide (Pentapharm, Basel, Switzerland), is preferred. Upon reaction with Factor Xa, a signal molecule P-nitroaniline is released, which may be conveniently measured by spectrophotometric determination at 405nm.

Other chromogenic indicator agents which are applicable with the present invention are available also. From the preceding disclosure it will be apparent to those skilled in the art that the signal moiety of the target indicator agent may be radiolabelled, preferably by tritium or carbon 14, and the signal molecule upon release can be isolated by exclusion chromatography, dialysis, immunoadsorption, or other convenient separation techniques. Radiolabelled indicator agents, while more cumbersome to use, have the advantage of greater sensitivity in those situations wherein unusually great sensitivity is needed.

It is contemplated within the scope of the present invention that the components of the Factor VIII:Ca assay may be available as a kit for the convenient and routine performance of a large number of such assays.

A kit for performing a Factor VIII:C assay on a sample comprising: a) a first vessel containing: a sufficient amount of Factor IXa to saturate all Factor VIII:Ca in said sample, a sufficient amount of a thrombin inhibitor to inhibit thrombin activity without affecting Factor Xa activity, a sufficient amount of phospholipid and calcium ion to facilitate the conversion of Factor X to Factor Xa; b) a second vessel containing a sufficient amount of Factor X to saturate the complex of Factors IXa and VIII:C and phospholipid; c) a third vessel containing a sufficient quantity of an indicator agent capable of reacting with Factor Xa, said quantity of indicator agent being sufficient to suppress Factor Xa side reactions. An optimal fourth vessel can be included with a quenching composition.

To optimize shelf life of the components of the kit it is desirable to lyophilize them in the aforementioned vessels. The said components may be readily reconstituted by adding water at the time assays are to be performed. The vessels containing assay components are readily adapted to automated assay equipment.

EXAMPLE 1. FACTOR VIII:Ca GENERATION IN THROMBOPLASTIN ACTIVATED PLASMA

Factor VIII:Ca generation is triggered by addition of 10 µl of a solution of $CaCl_2$ (167 mM) containing human brain thromboplastin (dilution 1/18) to 90 µl of defibrinated plasma. At various times aliquots of this mixture are taken for assay of the Factor VIII:Ca concentration. Each aliquot is diluted 100 fold in a cuvette containing Factor IXa (100 nM), phospholipid vesicles (20 µM, 20 mole-% phosphatidyl serine/80 mole-% phosphatidyl choline), $CaCl_2$ (5 mM) and -NAPAP (1 µM), in buffer at 37°C. After 10 seconds, Factor Xa chromogenic substrate ($CH_3OCO-D-CHG-Gly-Arg-pNA-AcOH$; Pentapharm, Basel Switzerland) is added to the cuvette so as to obtain a concentration of 400 µM. Factor X activation is started after another 10 seconds by addition of Factor X, to a concentration of 0.33 µM. Concentrations mentioned are final, i.e. they are obtained after all additions to the cuvette have been made. The course of absorbance at 405 nm is then measured continuously in time.

The build up of absorbance in time is governed by the following equation:

$$A(t). = A_0 + k1 \cdot [X_2]_0 \cdot t + k2 \cdot [VIII:Ca] \cdot t$$

The quadratic term which is a function of the Factor VIII:Ca concentration is estimated from the absorbance-time curve by way of a quadratic least squares fit procedure. From this the Factor VIII:Ca concentration is inferred, using a value for k2 of 1.164 mA/min$^2$/%VIII:ca, which has been determined previously.

Factor VIII:Ca concentrations at each time point, stated as a percentage of the total Factor VIII:C, are shown in the table below.

## TABLE 1

| Time (min) | [VIII:Ca] (%) |
|---|---|
| 0.50 | 0.7 |
| 1.00 | 2.9 |
| 1.25 | 10.1 |
| 1.50 | 44.1 |
| 1.75 | 73.9 |
| 2.00 | 62.1 |
| 2.25 | 49.7 |
| 2.75 | 39.8 |

## Claims

1. A kit for performing a Factor VIII:Ca assay on a sample comprising:
   a) a first vessel containing: a sufficient amount of Factor IXa to saturate all Factor VIII:Ca in said sample, a sufficient amount of a thrombin inhibitor to inhibit thrombin activity without affecting Factor Xa activity, a sufficient amount of phospholipid and calcium ion to facilitate the conversion of Factor X to Factor Xa;
   b) a second vessel containing a sufficient amount of Factor X to saturate the complex of Factors IXa and VIII:Ca and phospholipid; and
   c) a third vessel containing a sufficient quantity of an indicator agent capable of reacting with Factor Xa, said quantity of indicator agent being sufficient to suppress Factor Xa side reactions.

2. The kit of Claim 1 where Factors IXa and X are of bovine origin.

3. The kit of Claim 1 or 2 wherein the phospholipid is comprised of: between 5 to 40 mole-% phosphatidyl serine, 0 to 20 mole-% cholesterol and 50-90 mole-% of phosphotidyl choline.

4. The kit of Claim 1 or 2 wherein the phospholipid is comprised of 20 mole-% of phosphatidyl serine and 80 mole-% phosphatidyl choline.

5. The kit of any preceding Claim wherein the said indicator is selected from the class consisting of: enzymatic, radiometric, fluorescent and chromogenic indicators.

6. The kit of Claim 5 wherein said indicator is chromogenic.

7. A kit according to any preceding claim, comprising a fourth vessel containing a sufficient quantity of quenching agent to stop the conversion of Factor X to Factor Xa.

8. The kit of Claim 7 wherein said quenching agent is any substance capable of disrupting a protein-mediated chemical reaction.

9. The kit of Claim 8 wherein the quenching substance is a buffered solution of Tris, ethylenediaminetetracetic acid, sodium chloride and sodium azide.

10. A method for determining the concentration of blood coagulation Factor VIII:Ca in a fluid sample comprising:
    a) combining into a mixture said fluid sample with a sufficient amount of Factor IXa to saturate substantially all Factor VIII:Ca in said sample; a sufficient amount of thrombin inhibitor to inhibit thrombin activity without affecting Factor Xa activity in the presence of a sufficient amount of calcium ions and phospholipids to facilitate the conversion of Factor X to Factor Xa;
    b) adding a sufficient amount of Factor X to saturate the complex of Factors IXa and VIII:Ca and phospholipid;
    c) adding a sufficient quantity of an indicator agent capable of reacting with Factor Xa, whereby to release a signal molecule said quantity of indicator agent being sufficient to suppress Factor Xa side reactions; and

d) measuring the signal molecule.

11. The method of Claim 10, together with the further step of adding a quenching substance at a fixed point in time after the addition of Factor X to prevent the conversion of Factor X to Factor Xa.

12. The method of Claim 10 or 11 wherein Factors IXa and X are obtained from an animal source.

13. The method of Claim 10 or 11 wherein Factors IXa and X are obtained from a recombinant vector propagated in a host cell line.

14. The method of any one of Claims 10 to 13 wherein the indicator agent is selected from the class consisting of enzymatic, radiometric, fluorescent and chromogenic indicators.

15. The method of Claim 14 wherein said indicator is chromogenic.

16. The method of Claim 15 wherein said indicator is $CH_3OCO$-D-CHG-Gly-Arg-pNA-AcOH.

17. A method for indirectly determining the concentration of blood coagulation enzyme wherein the substrate for said enzyme is the zymogen of a second enzyme in a fluid sample comprising:
a) combining said blood coagulation enzyme, said zymogen of a second enzyme, and an indicator agent for said second enzyme in sufficient quantities to block all reactions of said second enzyme other than liberating a signal molecule from said indicator agent, and
b) measuring the signal molecule.

## Patentansprüche

1. Set zum Durchführen eines Faktor-VIII:Ca-Assays an einer Probe, das aufweist:
a) ein erstes Gefäß, das enthält: eine ausreichende Menge an Faktor IXa, um den gesamten Faktor VIII:Ca in der Probe zu sättigen, eine ausreichende Menge eines Thrombininhibitors, um die Thrombinaktivität zu inhibieren, ohne die Aktivität von Faktor Xa zu beeinträchtigen, eine ausreichende Menge an Phospholipid und Calciumionen, um die Umwandlung von Faktor X in Faktor Xa zu erleichtern;
b) ein zweites Gefäß, das eine ausreichende Menge an Faktor X enthält, um den Komplex aus Faktoren IXa und VIII:Ca und Phospholipid zu sättigen; und
c) ein drittes Gefäß, das eine ausreichende Menge eines Indikators enthält, der zur Reaktion mit Faktor Xa fähig ist, wobei die Indikatormenge ausreicht, um Nebenreaktionen von Faktor Xa zu unterdrücken.

2. Set nach Anspruch 1, wobei die Faktoren IXa und X vom Rind stammen.

3. Set nach Anspruch 1 oder 2, wobei das Phospholipid besteht aus: 5 bis 40 Mol-% Phosphatidylserin, 0 bis 20 Mol-% Cholesterin, und 50 bis 90 Mol-% Phosphatidylcholin.

4. Set nach Anspruch 1 oder 2, wobei das Phospholipid aus 20 Mol-% Phosphatidylserin und 80 Mol-% Phosphatidylcholin besteht.

5. Set nach einem der vorhergehenden Ansprüche, wobei der Indikator aus der Klasse ausgewählt ist, die aus folgendem besteht: enzymatischen, radiometrischen, fluoreszierenden und chromogenen Indikatoren.

6. Set nach Anspruch 5, wobei der Indikator chromogen ist.

7. Set nach einem der vorhergehenden Ansprüche, das ein viertes Gefäß aufweist, das eine ausreichende Menge an Quenchmittel enthält, um die Umwandlung von Faktor X in Faktor Xa abzubrechen.

8. Set nach Anspruch 7, wobei das Quenchmittel irgendeine Substanz ist, die fähig ist, eine Protein-vermittelte chemische Reaktion zu unterbrechen.

9. Set nach Anspruch 8, wobei die Quenchsubstanz eine gepufferte Lösung aus Tris, Ethylendiamintetraessigsäure, Natriumchlorid und Natriumazid ist.

10. Verfahren zum Bestimmen der Konzentration des Blutgerinnungsfaktors VIII:Ca in einer Fluidprobe, wobei das Verfahren die folgenden Schritte aufweist:

a) Vereinigen zu einem Gemisch: der Fluidprobe mit einer ausreichenden Menge an Faktor IXa, um im wesentlichen den gesamten Faktor VIII:Ca in der Probe zu sättigen; einer ausreichenden Menge an Thrombininhibitor, um die Thrombinaktivität zu inhibieren, ohne die Aktivität von Faktor Xa zu beeinträchtigen, in Gegenwart einer ausreichenden Menge an Calciumionen und Phospholipiden, um die Umwandlung von Faktor X in Faktor Xa zu erleichtern;

b) Zusetzen einer ausreichenden Menge an Faktor X, um den Komplex aus Faktoren IXa und VIII:Ca und Phospholipid zu sättigen;

c) Zusetzen einer ausreichenden Menge eines Indikators, der zur Reaktion mit Faktor Xa fähig ist, so daß ein Signalmolekül freigesetzt wird, wobei die Menge des Indikators ausreicht, um Nebenreaktionen von Faktors Xa zu unterdrücken; und

d) Messen des Signalmoleküls.

11. Verfahren nach Anspruch 10 gemeinsam mit dem weiteren Schritt des Zusetzens einer Quenchsubstanz zu einem bestimmten Zeitpunkt nach dem Zusetzen von Faktor X, um die Umwandlung von Faktor X in Faktor Xa zu verhindern.

12. Verfahren nach Anspruch 10 oder 11, wobei die Faktoren IXa und X aus einer tierischen Quelle erhalten werden.

13. Verfahren nach Anspruch 10 oder 11, wobei die Faktoren IXa und X von einem in einer Wirtszellinie fortgepflanzten rekombinanten Vektor erhalten werden.

14. Verfahren nach einem der Ansprüche 10 bis 13, wobei der Indikator aus der Klasse ausgewählt ist, die aus enzymatischen, radiometrischen, fluoreszierenden und chromogenen Indikatoren besteht.

15. Verfahren nach Anspruch 14, wobei der Indikator chromogen ist.

16. Verfahren nach Anspruch 15, wobei der Indikator $CH_3OCO$-D-CHG-Gly-Arg-pNA-AcOH ist.

17. Verfahren zum indirekten Bestimmen der Konzentration von Blutgerinnungsenzym, wobei das Substrat für das Enzym das Zymogen eines zweiten Enzyms in einer Fluidprobe ist, wobei das Verfahren die folgenden Schritte aufweist:

a) Vereinigen des Blutgerinnungsenzyms, des Zymogens eines zweiten Enzyms und eines Indikators für das zweite Enzym in ausreichenden Mengen, um sämtliche Reaktionen des zweiten Enzyms, die nicht das Freisetzen eines Signalmoleküls aus dem Indikator sind, zu blockieren, und

b) Messen des Signalmoleküls.

## Revendications

1. Trousse pour mettre en oeuvre un dosage du facteur VIII:C dans un échantillon, qui comprend :

a) un premier récipient contenant : une quantité suffisante du facteur IXa pour saturer la totalité du facteur VIII:Ca dans cet échantillon, une quantité suffisante d'un inhibiteur de thrombine pour inhiber l'activité de thrombine sans affecter l'activité du facteur Xa, une quantité suffisante de phospholipides et de l'ion calcium pour faciliter la conversion du facteur X en le facteur Xa ;

b) un deuxième récipient contenant une quantité suffisante du facteur X pour saturer le complexe des facteurs IXa et VIII:C et du phospholipide ; et

c) un troisième récipient contenant une quantité suffisante d'un agent indicateur capable de réagir avec le facteur Xa, cette quantité de l'agent indicateur étant suffisante pour supprimer les réactions secondaires du facteur Xa.

2. Trousse selon la revendication 1, dans laquelle les facteurs IXa et X sont d'origine bovine.

3. Trousse selon la revendication 1 ou 2, dans laquelle le phospholipide est constitué de 5 à 40 % en moles de phosphatidylsérine, de 0 à 20 % en moles de cholestérol et de 50 à 90 % en moles de phosphatidylcholine.

4. Trousse selon la revendication 1 ou 2, dans laquelle le phospholipide est constitué de 20 % en moles de phosphatidylsérine et de 80 % en moles de phosphatidylcholine.

5. Trousse selon l'une quelconque des revendications précédentes, dans laquelle l'indicateur est choisi parmi l'ensemble comprenant les indicateurs enzymatiques, radiométriques, fluorescents et chromogènes.

6. Trousse selon la revendication 5, dans laquelle l'indicateur est chromogène.

7. Trousse selon l'une quelconque des revendications précédentes, qui comprend un quatrième récipient contenant une quantité d'un agent d'extinction suffisante pour arrêter la conversion du facteur X en le facteur Xa.

8. Trousse selon la revendication 7, dans laquelle l'agent d'extinction est toute substance à même d'interrompre une réaction chimique à médiation protéique.

9. Trousse selon la revendication 8, dans laquelle la substance d'extinction est une solution tamponnée, de Tris, d'acide éthylènediaminetétraacétique, de chlorure de sodium et d'azothydrure de sodium.

10. Procédé pour déterminer la concentration du facteur de coagulation sanguine VIII:Ca dans un échantillon de fluide, qui consiste :

a) à combiner en un mélange l'échantillon de fluide à une quantité suffisante du facteur IXa pour saturer pour ainsi dire la totalité du facteur VIII:Ca se trouvant dans l'échantillon ; une quantité suffisante d'un inhibiteur de thrombine pour inhiber l'activité de thrombine sans affecter l'activité du facteur Xa en présence d'une quantité suffisante d'ions calcium et de phospholipides pour faciliter la conversion du facteur X en le facteur Xa ;

b) à ajouter une quantité du facteur X suffisante pour saturer le complexe des facteurs IXa et VIII:Ca et du phospholipide ;

c) à ajouter une quantité suffisante d'un agent indicateur capable de réagir avec le facteur Xa, pour libérer une molécule signal, la quantité de l'agent indicateur étant suffisante pour supprimer les réactions secondaires du facteur Xa ; et

d) à doser la molécule signal.

11. Procédé selon la revendication 10, couplé à l'étape supplémentaire consistant à ajouter une substance d'extinction à un instant donné après addition du facteur X, pour empêcher la conversion du facteur X en le facteur Xa.

12. Procédé selon la revendication 10 ou 11, dans lequel les facteurs IXa et X sont obtenus d'une source animale.

13. Procédé selon la revendication 10 ou 11, dans lequel les facteurs IXa et X sont obtenus à partir d'un vecteur recombinant propagé dans une lignée cellulaire hôte.

14. Procédé selon l'une quelconque des revendications 10 à 13, dans lequel l'agent indicateur est choisi parmi l'ensemble comprenant les indicateurs enzymatiques, radiométriques, fluorescents et chromogènes.

15. Procédé selon la revendication 14, dans lequel l'indicateur est chromogène.

16. Procédé selon la revendication 15, dans lequel l'indicateur est $CH_3OCO$-D-CHG-Gly-Arg-pNA-AcOH.

17. Procédé de détermination indirecte de la concentration de l'enzyme de coagulation du sang, où le substrat de l'enzyme est le zymogène d'une deuxième enzyme, dans un échantillon de fluide, qui consiste :

a) à combiner ladite enzyme de coagulation sanguine, ledit zymogène d'une deuxième enzyme, et d'un agent indicateur de ladite deuxième enzyme, en des quantités suffisantes pour bloquer toutes les réactions de ladite deuxième enzyme autres qu'une libération d'une molécule signal à partir du dit agent indicateur, et

b) à doser la molécule signal.